# EUROPEAN PATENT SPECIFICATION

(11) **EP 2 598 137 B1**
(45) Date of publication and mention of the grant of the patent: **06.07.2022**
(21) Application number: 10855255.5
(22) Date of filing: 29.07.2010
(51) Int. Cl.: A61K 31/00, A61K 33/40, A61K 31/255, A61K 31/327, A61P 31/04

(54) **METHOD OF TREATING ANIMAL DRINKING WATER WITH INTERVAL DOSING OF A BIOCIDE**
VERFAHREN ZUR BEHANDLUNG VON TRINKWASSER FÜR TIERE MIT INTERVALLDOSIERUNG EINES BIOZIDS
PROCÉDÉ DE TRAITEMENT DE L'EAU DE BOISSON D'UN ANIMAL, AVEC INTRODUCTION DOSÉE PÉRIODIQUE D'UN BIOCIDE

(43) Date of publication of application: 05.06.2013
(60) Divisional application: 13169190.9
(73) Proprietor: Ecolab USA Inc., St. Paul, MN 55102 (US)
(72) Inventor: VERKAAR, Edward L.C., NL-5991 L.W. Baarlo (NL); HENDRIKS, Erik, NL-4003 GE Tiel (NL); HILGREN, John D., Shoreview Minnesota 55126 (US)
(74) Representative: Godemeyer Blum Lenze Patentanwälte Partnerschaft mbB - werkpatent
(86) International application number: PCT/IB2010/053464
(87) International publication number: WO 2012/014016

(56) References cited:
- WO-A1-01/52827
- WO-A2-2009/152332
- US-A1- 2002 115 719
- US-A1- 2002 173 545
- US-A1- 2004 209 955
- US-A1- 2005 118 940
- US-B1- 6 627 657
- BARNHART, E. T. ET AL.: 'Evaluation of potential disinfectants for preslaughter broiler crop decontamination' POULTRY SCIENCE vol. 78, 1999, pages 32 - 37, XP001079560

## Description

### Background

The present invention relates to a biocide for use in prophylaxis of infection, in particular for use in reducing and/or controlling the microintestinal population in the gastrointestinal tract of living animals.

The consumption of fresh poultry has been associated epidemiologically with outbreaks of gastroenteritis due to *Campylobacter jejun*i or *Salmonella spp.* in both developed and undeveloped countries. Chicken meat is the second most common food item associated with outbreaks of *Salmonella* or *Campylobacter* in Europe and Northern America. Most cases are sporadic and dependent on many factors such as age, pregnancy, and immune deficiency to name a few. There is considerable evidence to suggest that *Campylobacter* and/or *Salmonella* infections are linked with handling of, or cross contamination with, live or dead poultry. Several other sources have been linked to Campylobacteriosis or Salmonellosis as well. One of the main sources identified with such an outbreak is water or the drink-water system offered to the animals.

According to EU regulation EU178/2002, drinking or soaking water may be regarded as feed. As of 1 January 2006, this regulation is part of the EU183/2005. This regulation clearly states that drinking water should be "suitable for the animals concerned." According to this regulation, water, treated with decontaminants may be used. However, it is clear that not all potential decontaminants in drinking water will produce the required or desired results. There are a number of different chemicals used in poultry drinking water. Some chemicals are used for aggressive cleaning (when no birds are present) while others are used to maintain water quality (when birds are present). Chemicals such as chlorine, organic acids and/or hydrogen peroxide are examples of products applied continuously for a shorter or longer term to eradicate biofilms or lower the overall microbiological load in animal drinking water. Many chemicals can be used for either application such as ammonia hydroxide, organic acids, hydrogen peroxide, iodine, sodium bisulfate, quaternary ammonium, and potassium peroxymonosulfate to name a few.

Currently, there are a number of animal drinking water intervention options which are, due to the low application concentration, marginally or completely ineffective. Incorporating high concentrations of biocide in the drinking water may be detrimental for the animal health or physical condition and as such is unsuitable to use in practice.

Drinking water lines in poultry and other animal farms contain considerable amounts of biofilm build up from bacteria, yeasts and molds. "Biofilm" is a generic term used to describe the organic, primarily polysaccharide, layer that builds up on the inner surfaces of the water supply system due to microbial action. *E. coli*, *Salmonella* and *Campylobacter* are some of the organisms capable of forming biofilms. Regardless of the source, the presence of biofilms has been shown to significantly enhance the survival times of microorganisms, the extent of the effect being influenced by the genus and strain of the microorganism. The presence of biofilms has also been found to reduce the efficacy of sanitizers. Although microorganisms commonly causing biofilms are not specifically pathogenic in nature, it is clear that they form an outstanding source and ground for pathogenic ubiquitous microorganisms.

Biofilm loading may also increase the sedimentation of mineral deposits. These mineral deposits affect water output and pressure. Moreover, mineral deposits in or near the nipples may cause leaking of the nipples as the bullets cannot close. Leaking nipples may cause wet beddings which will negatively affect a bird's health status and the formation of ammonia in contact with the excrements. Both issues are described in more detail below and have a negative impact on an animal's health.

As is well known in the art, poultry drinking water is provided by water lines connected to drinker cups and/or nipples. The drinker cups have a white ball attached to a nipple, which fill the drinker cup with water. Drinker nipples are held in place by water pressure in the water lines, so that they are normally closed. The bird pushes on the nipple to open the nipple to allow water to drip out. Scale formation has been a major problem in the past in connection with drinker cups and water lines. The valve mechanism includes a seat and ball, and scale forms between the seat and the ball, which prevents the nipple from closing, so that the drinking cup leaks. Leaking drinking cups cause several problems. First, the poultry are stacked in cages, so that if a drinking cup at the top of the stack is leaking, poultry below will get wet. Secondly, the manure on the ground will get wet which is undesirable since this produces ammonia and which is hazardous to the poultry and maintenance personal. Also, the poultry does not consume the water fast enough and the remaining water becomes stagnant with a bad odor or leaks and drips on the poultry litter. The water in the drinker cups can also become contaminated with feces and bacterial growth. It has been documented that chlorine should not be used to clean the drinker cups because the poultry does not like the smell. The poultry will back off drinking water from the drinking cups and drink poor quality water elsewhere, which can make them sick, and causes them to eat less. The poultry gut flora gets destroyed so that the poultry cannot absorb nutrients correctly. The poultry can get secondary infections and suffer from dehydration. All of these health problems result in poorer quality poultry, and poultry with higher mortality rates.

The nipples may also become a source when an infected beak contacts the nipple. If a nipple cannot close properly, bacteria (connected with feedstuffs) may enter the water-line and form biofilms. At low water pressure these and other bacteria may build biofilms from the nipple along the water line.

Pathogenic bacteria from exogenous sources (e.g. *Campylobacter* or *Salmonella*) may enter a flock by rodents, wild bird excrement or insects. Bacteria are capable of introducing a wide spreading infection inside a flock by picking into infected excrements; a bird's gut may become easily infected and subsequently infect multiple other members of the flock. A spread may take place in the time frame of several hours.

Several studies show increased resistance towards biofilms with medium to lower concentrations of biocides. In order to be effective, it is necessary to use higher concentrations of biofilm removing agents. However, as already suggested, the applied agent may lead to sensory disapproval by the animals and result in decreased water uptake. It is preferable to avoid negatively impacting water expenditure since it affects food conversion ratio and growth of the animals.

Previous studies of adding organic acids in animal drinking water (formic acid, acetic acid) or acidifying animal feedstuff (by fermentation, adding lactic, formic or acetic acid) showed that animals had difficulties with adapting to the organoleptic differences. Although the effect was temporary, it appears that animals need to be trained into using these products which in turn will positively affect the feed conversion ratio.

The veterinary effect of biofilms and/or bacteria towards the flocks is less obvious. It is not clear if the endogenous biofilms and subsequent bacteria are harmful for birds. First, it is clear that exogenous bacteria will have less chance to colonize and grow in the absence of biofilms. Second, in principle *Pseudomonads* are opportunistic pathogens, meaning that under certain periods of immuno-incapacities or stress, diseases may be triggered. Therefore, high loads of indicator organisms as total plate counts and enterobacteriaceae (coliforms) may be closely related to hygiene and health status of the animals.

Current practices in confined animal production include placing the animals through a feed withdrawal period for 4 to 18 hours prior to transportation to the slaughter facility. It is during this feed withdrawal period that poultry begin to peck at the bedding material that, by the time the birds are ready to be shipped to the abattoir, becomes severely contaminated with feces. If these feces contain pathogenic bacteria such as *Salmonella* or *Campylobacter,* these organisms will quickly colonize organ lumen areas throughout the digestive tract of the birds, especially in the upper GI tract.

There is a need for a method of controlling the microbial population in the gastrointestinal tract of animals. Preferably the method would include non-invasive, oral administration of a biocidal compound or composition that could be added to drinking water, feed/foodstuffs, or other ingestible forms. More preferably, the presence of the compound or composition in water or feed/foodstuffs would not negatively impact the consumption of the water or feed/foodstuffs by the animals. It would also be desirable if the compound or composition would decompose or form by-products that are harmless to the animal and, in the case of animals to be slaughtered for consumption, either the byproducts are not partitioned in or on the edible portions of the carcass or are harmless to those consuming the meat of the animal.

WO 01/52827 A1 describes a method to control microbial populations in the gastrointestinal tract of animals comprising the step of administering an effective amount of a peracid compound to an animal.

### Summary

The present invention relates to a biocide comprising peracid for use in prophylaxis of infection, in particular for use in reducing and/or controlling the microbial population in the gastrointestinal tract of living animals, wherein the biocide is orally administered in an effective amount to an animal by interval dosing, wherein the interval dosing comprises administering orally between about 10 to about 60 ppm biocide for a time period of between about 5 and 30 minutes supplied at between about every 45 minutes to about every 4 hours. Control or prevention of microbial growth is achieved by intervally administering a biocide in an aqueous stream which is subsequently consumed orally by the animal. The interval administration is created by providing the biocide-treated medium, such as water or foodstuff, followed by administration of an untreated medium and repeating indefinitely. In an embodiment, the biocide is percarboxylic acid or a mixture of percarboxylic acids and sulfuric acid. In an embodiment the biocide is intermittently provided in an aqueous stream which is subsequently consumed orally by the animal with each biocide dosing followed by a biocide-free aqueous stream.

The method of the invention is unexpectedly effective in preventing death of animals in captivity due to infection. The method of the invention is unexpectedly effective in increasing the feed conversion rate in food-producing animals such as in egg laying hens and dairy cattle. The method of the invention is also unexpectedly effective in preventing and/or controlling the growth of unwanted microorganisms within body cavities and digestive tract of animals prior to the slaughter of the animal. The formulation exerts its decontaminating effect beyond the oral cavity and into the intestinal regions of the animal. The intermittent oral consumption of appropriately formulated biocides such as percarboxylic acids followed by no biocide causes an unexpectedly high level of internal decontamination of the animal. Moreover, the intermittent oral consumption of biocides according to the invention results in an increased feed conversion.

The invention is particularly efficacious in reducing the growth of unwanted microorganisms in egg producing hens, dairy cattle, and during the feed withdrawal period that usually extends 0.1 to 1.5 days before slaughter.

The invention effectively combines a high degree of internal antimicrobial efficacy with a high degree of palatability by the animal. The formulation can be safely ingested by animals while imposing no environmental incompatibility or ill health. The consumed percarboxylic formulations break down inside an animal's body to result in harmless end products.

A method of intermittently or intervally administering a biocide is provided. A method for controlling microbial populations in the gastrointestinal tract of living animals is also provided. The methods include preparing an effective amount of a biocide compound and providing it to an animal by dosing in intervals. The biocide may include a peracid including but not limited to a peroxygenated carboxylic acid such as performic, peracetic, perproprionic, peroxyheptanoic, peroxynonanoic, perlauric, monoperglutaric, diperglutaric, succinylperoxide, derivatives of perbenzoic acid, magnesium salt of peroxyphthalate, benzoyl peroxide, t-butylhydroperoxide, perlactic, percitric, perbutyric, peroctanoic, and perglycolic. In an embodiment sulfuric acid is administered along with the peracid. The method of the invention is effective in reducing the population of pathogens, pathogenic microorganisms, and microbes including but not limited to *Salmonella, Campylobacter*, *E. Coli*, *Listeria*, and *Helicobacter.*

In an embodiment the biocide is provided in drinking water.

### Brief Description of the Drawings

Figure 1 is a graphical depiction of the mean daily intake of water by poultry offered two different biocides, an intervally dosed biocide, and a control.
Figure 2 is a graphical depiction of the summarized daily intake of water by poultry shown in Figure 1. The birds offered the intervally dosed biocide had nearly similar intake as those offered the same biocide constantly.
Figure 3 is a graphical depiction of daily mortality rate (deaths per 10,000 birds) of poultry constantly offered one of two biocides, an intervally dosed biocide, and a control.
Figure 4 is a graphical depiction of the summarized daily mortality rate (deaths per 10000 birds) shown in Figure 3. The birds offered the intervally dosed biocide had a lower death rate as compared to all other birds. Figure 5 is a graphical depiction of the daily egg production of poultry constantly offered one of two biocides, an intervally dosed biocide, and a control. The birds offered the intervally dosed biocide had a substantially higher egg production as compared to all other birds.

### Detailed Description of Some Preferred Embodiments

The present invention refers to a biocide comprising peracid for use in prophylaxis of infection, in particular for use in reducing and/or controlling the microbial population in the gastrointestinal tract of living animals, wherein the biocide is orally administered in an effective amount to an animal by interval dosing, wherein the interval dosing comprises administering orally between about 10 to about 60 ppm biocide for a time period of between about 5 and 30 minutes supplied at between about every 45 minutes to about every 4 hours. The method comprises the step of orally administering an effective amount of a peracid to an animal at intervals.

The term "animals" as used herein means vertebrate animals, including but not limited to poultry, fish, cattle, swine, goats, lambs, dogs, cats, rodents, rabbits, birds, deer, non-human primates, and others.

Antimicrobial terms such as bacteria-"-cidal" or bacteria"-static" describe the degree of efficacy of an agent. The official laboratory protocols for measuring this efficacy are important considerations for understanding the relevance of antimicrobial agents and compositions. Antimicrobial compositions may affect two kinds of microbial cell damage. The first is a truly lethal, irreversible action resulting in complete microbial cell destruction or incapacitation referred to herein as "bactericidal." The second type of cell damage is reversible, such that if the organism is rendered free of the agent, it can again multiply and is referred to herein as "bacteriostatic." A sanitizer and a disinfectant are, by definition, agents which provide antibacterial or bactericidal activity. In contrast, a preservative is generally described as an inhibitor or bacteriostatic composition.

The term "biocide" as used herein is any bacteriostatic or bactericidal agent useful to control or prevent the growth of microorganisms. In an embodiment a biocide is a compound that is generally considered safe for human consumption. A biocide includes but is not limited to organic and/or inorganic compounds. A biocide for purposes of the invention can be provided as a single composition or a combination of more than one composition or may be provided as a reaction product of any number of chemical compositions.

The term "substantially free" may refer to any component that the composition of the invention lacks or mostly lacks. When referring to "substantially free" it is intended that the component is not intentionally added to compositions of the invention. Use of the term "substantially free" of a component allows for trace amounts of that component to be included in compositions of the invention because they are present in another component. However, it is recognized that only trace or de minimus amounts of a component will be allowed when the composition is said to be "substantially free" of that component. It is understood that the invention is "substantially free" of all chemicals and compositions not expressly named herein. It is also understood that by expressly naming a chemical or composition herein the inventors may intend to excise it from the invention.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

As used herein, weight percent (wt-%), percent by weight, % by weight, and the like are synonyms that refer to the concentration of a substance as the weight of that substance divided by the total weight of the composition and multiplied by 100. All percentages provided herein are provided on a weight percent basis unless otherwise noted.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It has surprisingly been found that a biocide provided in interval doses to vertebrate animals is at least as effective at controlling, reducing, or preventing microbial growth in the intestinal tracts as compared to providing the same biocide constantly. More importantly, it has been discovered that providing a biocide in interval doses produces a greater feed to product weight conversion rate than providing the same biocide constantly or as compared to a biocide-free control. In the case of egg-producing hens, this results in higher egg production per amount of feed. That is, the intermittent administration to an animal of a biocide in a carrier followed by administration to the animal of the untreated carrier (biocide-free) and then repeating the dosing is at least equal to and often superior to the effects of constant dosing of the biocide.

A "carrier" for purposes of this invention includes water, foodstuffs, and any other medium commonly provided orally to vertebrate animals.

If the biocide is delivered to a food animal for the purpose of preventing contamination of meat surfaces during slaughter, then it is preferred that the biocide is administered over a period just preceding slaughter. Such a time period might include 0.01 days up to about 5 days preceding slaughter, 0.1 to 3 days, or about 0.5 to 2 days.

In an embodiment the biocide is a peracid formed by the reaction product of the combination of an organic acid having one to eight carbon atoms and an inorganic acid and an inorganic peroxide compound. In another embodiment sulfuric acid is added in combination with the peracid. Without being bound by theory, it is believed that the combination of peracetic acid and sulfuric acid provides the appropriate pH to remove mineral deposits on which biofilms might attach and grow, to lower optimal pH in order to inhibit growth of most biofilm forming micro-organisms.

The peroxide of many organic acids have the attributes of hydrogen peroxide. That is, they have effective germicidal and sterilizing capabilities, benign decomposition products, and infinite water solubility but with greater lipid solubility and freedom from deactivation by catalase and peroxidases. The peroxide of acetic acid, peroxyacetic acid, or peracetic acid (PAA) is an effective biocide with no toxic residues and is widely used as a surface disinfectant in the food processing industry. PAA is a more potent antimicrobial agent than hydrogen peroxide alone, being rapidly active at low concentrations against a wide spectrum of microorganisms. It is sporicidal even at low temperatures and remains effective in the presence of organic matter. As a weak acid it is more active on the acid side but is germicidal with higher concentration in the alkaline range. Like hydrogen peroxide (HP), it is useful both in solution and as a vapor. These properties make it a remarkably valuable compound when practicing methods of the invention.

Peroxides in general are high-energy-state compounds, and as such can be considered thermodynamically unstable. PAA is considerably less stable than HP. A solution of 40% PAA loses 1 to 2% of its active ingredients per month, as compared with HP (30 to 90%), which loses less than 1% per year. The decomposition products of PAA are acetic acid, HP, oxygen, and water. Dilute PAA solutions are even more unstable with a 1% solution losing half its strength through hydrolysis in 6 days.

In an embodiment of the invention it may therefore be desirable to mix the components of the peracid or peroxygen solution at or near the location at which the biocidal solution is to be administered. The individual components may therefore be prepared commercially in separate containers or matched together such that the number of components for the final formulation is minimized but the stability of the precursor solutions is maximized. PAA is produced by the reaction of acetic acid or acetic anhydride with HP in the presence of sulfuric acid, which acts as a catalyst.

The oxidation of organic carboxylic acids with hydrogen peroxide and an inorganic acid catalyst is another known method for the preparation of peroxy acids. A commonly known catalyst is concentrated sulfuric acid. The reaction is an equilibrium reaction and is driven to the right by removal of water or by the use of excess reagents. For aromatic R a desirable catalyst is methanesulfonic acid, which can be also used as the solvent.

Peroxygenated carboxylic acids have been shown to have excellent antimicrobial activity and have found utility in disinfecting diverse surfaces. These peroxy carboxylic acids are only moderately stable in aqueous solutions and therefore are most effective when used soon after the solutions are made. Examples of peroxygenated carboxylic acids include but are not limited to the following: performic, peracetic, perproprionic, peroxyheptanoic, peroxynonanoic, perlauric, monoperglutaric, diperglutaric, succinylperoxide, derivatives of perbenzoic acid, magnesium salt of peroxyphthalate, benzoyl peroxide, t-butylhydroperoxide, perlactic, percitric, perbutyric, peroctanoic, and perglycolic. Peroxygenated carboxylic acids are often known as, or referred to, by peracid compounds, peroxygen compounds, peroxo compounds and peroxides of organic acids.

Among other constituents, the invention may comprise a carboxylic acid. Generally, carboxylic acids have the formula R--COOH wherein the R may represent any number of different groups including aliphatic groups, alicyclic groups, aromatic groups, heterocyclic groups, all of which may be saturated or unsaturated as well as substituted or unsubstituted. Carboxylic acids also occur having one, two, three, or more carboxylic groups.

Carboxylic acids have a tendency to acidify aqueous compositions in which they are present as the hydrogen atom of the carboxyl group is active. Therefore, the carboxylic acids may appear as an anion in solution. The carboxylic acid constituent within the present composition when combined with aqueous hydrogen peroxide generally functions as an antimicrobial agent as a result of the presence of the active hydrogen atom. Moreover, the carboxylic acid constituent within the invention maintains the composition at an acidic pH.

Carboxylic acids which are generally useful in the process of the invention are those which comprise percarboxylic acids. Percarboxylic acids generally have the formula R(CO₃ H)ₙ, where R is an alkyl, arylalkyl, cycloalkyl, aromatic or heterocyclic group, and n is one, two, or three, and named by prefixing the parent acid with peroxy.

Peracid powder disinfectants also useful in this invention can be derived from water plus mixtures of organic acid reservoirs (e.g., anhydrides, amides, and esters) added to hydrogen peroxide reservoirs (e.g., sodium peroxide).

While peroxy carboxylic acids are not very stable, their stability generally increases with increasing molecular weight. Thermal decomposition of these acids may generally proceed by free radical and nonradical paths, by photodecomposition or radical-induced decomposition, or by the action of metal ions or complexes. Percarboxylic acids may be made by the direct, acid catalyzed equilibrium action of 30-98 wt. % hydrogen peroxide with the carboxylic acid, by autoxidation of aldehydes, or from acid chlorides, and hydrides, or carboxylic anhydrides with hydrogen or sodium peroxide.

Percarboxylic acids useful in this invention include peracetic acid, perpropionic acid, perbutyric acid, peroctanoic acid, perglycolic acid, perglutaric acid, persuccinic acid, perlactic acid, percitric acid, perdecanoic acid or mixtures thereof. These percarboxylic acids have been found to provide good antimicrobial action with good stability in aqueous streams.

In addition to peracetic, peroctanoic and perdecanoic, particularly preferred percarboxylic acids include perpropionic, perbutyric, perglycolic, perlactic and percitric acids.

The invention also uses a combination of peracetic acid with other percarboxylic acids, preferably, those named above and particularly, peroctanoic acid. This combination of percarboxylic acids has been found to provide preferred antimicrobial efficacy and stability in the presence of high organic loads. Generally, within the biocide, the concentration of, for example, peroctanoic acid may range from about 10 wt-% to 90 wt-% and preferably from about 10 wt-% to 20 wt-%. The concentration of peracetic acid may range from about 10 wt-% 90 wt-% and preferably from about 80 wt-% to 90 wt-%.

The invention also uses peracetic acid. Peracetic acid is a peroxy carboxylic acid having the formula, CH₃ COOOH. Generally, peracetic acid is a liquid having an acrid odor at high concentrations and is freely soluble in water, alcohol, ether, and sulfuric acid. Peracetic acid may be prepared through any number of means known to those of skill in the art including preparation from acetaldehyde and oxygen in the presence of cobalt acetate. A 50% solution of peracetic acid may be obtained by combining acetic anhydride, hydrogen peroxide and sulfuric acid. Other methods of formulation of peracetic acid include those disclosed in U.S. Pat. No. 2,833 813.

In an embodiment, the invention uses perlactic acid, CH₃CH(OH)COOOH.

Generation of the peroxy acids may be accomplished in various manners known in the art. Specifically, the peroxy acids may be formed through the use of peroxy acid concentrate compositions. In such a case, the percarboxylic acid may either be generated naturally or through the combination of a hydrogen peroxide concentrate together with a carboxylic acid concentrate at the site of use such as that process which is disclosed in Lokkesmoe et al, U.S. Pat. No. 5,122 538. Furthermore, the peroxy acids may be formed by the methods disclosed in Lokkesmoe et al., U.S. Pat. No. 5,674 538.

### Hydrogen Peroxide

The biocide of the invention may optionally comprise a hydrogen peroxide constituent. Hydrogen peroxide in combination with the percarboxylic acid provides a surprising level of antimicrobial action against microorganisms despite the presence of skin, tissue and intestinal contents, mucosa materials and membrane materials and sediment. Additionally, hydrogen peroxide may provide an effervescent action which may irrigate any surface to which it is applied. An additional advantage of hydrogen peroxide is that combinations of perlactic acid and hydrogen peroxide result in lactic acid, water, and oxygen upon decomposition all of which are food product compatible.

Generally, the concentration of hydrogen peroxide within the composition used in the process of the invention ranges from about 1 weight percent to about 50 weight percent, preferably from about 3 weight percent to about 40 weight percent, and most preferably from about 5 weight percent to about 30 weight percent. This concentration of hydrogen peroxide is most preferred as providing an optimal antimicrobial effect. These concentrations of hydrogen peroxide may be increased or decreased while still remaining within the scope of the invention.

### Additives

The biocide of the invention may also comprise any number of additives e.g. stabilizing agents, wetting agents, as well as growth factors, growth stimulants, vitamins, mineral supplements, antibiotics, drugs, and other nutrients that aid the growth and health of the animal receiving the formulation.

Stabilizing agents may be added to the composition of the invention to stabilize the percarboxylic and hydrogen peroxide formulation and prevent the premature oxidation of this constituent within the composition of the invention. Chelating agents or sequestrants generally useful as stabilizing agents include: alkyl diamine polyacetic acid-type chelating agents such as EDTA (ethylene diamine tetra acetate tetra sodium salt), acrylic and polyacrylic acid-type stabilizing agents, phosphonic acid, phosphonate-type chelating agents among others.

Preferable sequestrants include: phosphonic acids phosphonate salts including 1-hydroxy ethyldene-1, 1-diphosphonic acid (CH₃ C(PO₃ H₂)₂OH), amino[tri(methylene phosphonic acid)]([CH₂ PO₃ H₂ ]₂ (ethylene diamine[tetra methylene-phosphonic acid), 2-phosphene butane-1, 2, 4-tricarboxylic acid, as well as the alkyl metal salts, ammonium salts, or alkyloyl amine salts, such as mono, di, tri, or tetra-ethanolamine salts. The stabilizing agent is used in a concentration ranging from about 0 weight percent to about 20 weight percent of the composition, preferably from about 0.1 weight percent to about 10 weight percent of the composition, and most preferably from about 0.2 weight percent to 5 weight percent of the composition.

Also useful in the composition of the invention are wetting and defoaming agents. Wetting agents function to increase the penetration activity of the antimicrobial composition of the invention. Wetting agents which may be used in the composition of the invention include any of those constituents known within the art to raise the surface activity of the composition of the invention.

Along these lines surfactants, and especially nonionic surfactants, may also be useful in the present invention. Nonionic surfactants which may be useful in the present invention are those which comprise ethylene oxide moieties, propylene oxide moieties, as well as mixtures thereof, and ethylene oxide-propylene oxide moieties in either heteric or block formation. Additionally useful in the present invention are nonionic surfactants which comprise alkyl ethylene oxide compounds, alkyl propylene oxide compounds, as well as mixtures thereof, and alkyl ethylene oxide-propylene oxide compounds where the ethylene oxide-propylene oxide moiety is either in heteric or block formation. Further useful in the present invention are nonionic surfactants having any mixture or combination of ethylene oxide-propylene oxide moieties linked to a alkyl chain where the ethylene oxide and propylene oxide moieties may be in any randomized or ordered pattern and of any specific length. Nonionic surfactants useful in the present invention may also comprise randomized sections of block and heteric ethylene oxide propylene oxide, or ethylene oxide-propylene oxide.

Generally, the concentration of nonionic surfactant used in the invention may range from about 0 wt-% to about 5 wt-% of the composition, preferably from about 0 wt-% to about 2 wt-% of the concentrate composition, and most preferably from about 0 wt-% to about 1 wt-% of the composition.

The composition used in the process of the invention may also contain additional ingredients as necessary to assist in defoaming.

Generally, defoamers which may be used in accordance with the invention include silica and silicones; aliphatic acids or esters; alcohols; sulfates or sulfonates; amines or amides; halogenated compounds such as fluorochlorohydrocarbons; vegetable oils, waxes, mineral oils as well as their sulfated derivatives; fatty acid soaps such as alkali, alkaline earth metal soaps; and phosphates and phosphate esters such as alkyl and alkaline diphosphates, and tributyl phosphates among others; and mixtures thereof.

Especially preferable, are those antifoaming agents or defoamers which are of food grade quality given the application of the process of the invention. To this end, one of the more effective antifoaming agents comprises silicones. Silicones such as dimethyl silicone, glycol polysiloxane, methylphenol polysiloxane, trialkyl or tetralkyl silanes, hydrophobic silica defoamers and mixtures thereof may all be used in defoaming applications. Commercial defoamers commonly available include silicones such as Ardefoam^{™} from Armour Industrial Chemical Company which is a silicone bound in an organic emulsion; Foam Kill^{™} or Kresseo^{™} available from Krusable Chemical Company which are silicone and non-silicone type defoamers as well as silicone esters; and Anti-Foam^{®} and DC-200^{®} from Dow Corning Corporation which are both food grade type silicones among others. These defoamers are generally present at a concentration range from about 0 wt-% to 5 wt-%, preferably from about 0 wt-% to 2 wt-%, and most preferably from about 0 wt-% to about 1 wt-%.

### Food Agents

The formulation can contain flavoring agents, buffering agents and food preservatives: such as ascorbic acid, sorbic acid, citric acid, glutaric acid, phosphoric acid, and malic acid. These additives improve palatability and taste, thereby promoting consumption by the animals. These agents may also improve overall health and nutrition of the animals, as well as promoting further microbial destruction. Mineral salts may also be added. Glucose and sugars may also be added.

### Generation of Peroxy Acids

The process of the invention may also be initiated through the use of peroxy acid concentrate compositions. In such a case, the percarboxylic acid may either be generated naturally or through the combination of a hydrogen peroxide concentrate together with a carboxylic acid concentrate at the site of use such as that process which is disclosed in Lokkesmoe et al, U.S. Pat. No. 5,122,538, issued Jun. 16, 1992.

### Competitive Exclusion

The application of microorganisms as competitive exclusion microflora for the reduction of pathogen colonization in poultry has been discovered. Competitive exclusion microorganisms including *Clostridium* spp., *Streptococcus faecalis*, *Bifidobacterium* spp., and *Bacteroides hypernegas* have been examined. Furthermore, preparations containing several strains of single species, such as *Bacteroides* spp., *Bifidobacterium* spp., and *Escherichia* spp. have also been evaluated. Competitive exclusion diminish the populations of gram-negative enteropathogenic bacteria such as *Campylobacter* and *Salmonella.*

The invention can also be applied in tandem with the addition of competitive exclusion microorganisms to the animal. The peroxygen formulation is added to destroy target pathogens, e.g., human enteropathogenic bacteria capable of colonizing poultry. Of particular interest are *Salmonella* and *Campylobacter* species. Competitive exclusion microorganisms can be introduced to the intestinal tract subsequently, allowing sufficient time for the active disinfection ingredients to dissipate. The intestines, now harboring reduced numbers of microbial pathogens are more capable of being colonized by the exclusion organisms.

Both peroxygen compounds and competitive exclusion microorganisms can be administered by oral or nasal gavage, in drinking water, in feed/foodstuffs, by spraying newly born animals or newly hatched chicks with an aqueous suspension, or a combination of the above. This combined treatment would preferably be performed early and as frequently as possible.

### Interval Dosing

A mechanical system useful for practicing the method of the invention may include the following. A buffer tank including a buffer containing the diluted concentrated biocide (up to about 30 ppm by weight total product) and an electric pump to fill the buffer tank. The tank may be connected to the water line directly. A first cycle commences with a chemistry input at a predetermined amount time. During this stage a 'bulb' or "dose" of concentrate will enter the drinking line. At the end of the second stage, the valve of the buffer tank is closed and at a predetermined time-interval, the general water valve remains closed and the water level is expected to drop. A third stage is triggered at a predetermined time and the drinking line is filled with potable water. In this stage, all residual peracetic acid is flushed. After this stage, the cycle recommences. The skilled artisan will recognize that chemistry or biocide dosing times, water abstinence time and potable water rinse times are related to weight, age, temperature and species of the animal. The amount of water per animal is not only highly related to the latter parameters but may differ on a daily to weekly basis and should are adjusted according to the current condition in a farm. A drinking water line should never become completely dry.

Instead of cycles related to time triggers, an automated system may be constructed based on oval flow water meters that detect flow rate and according to flow rate inject correct amounts of chemistry or biocide. The system may be fitted with a centrifugal pump that turns off when the pressure of the line system exceeds a threshold. A valve may be switched on if the pressure drops to a certain point and chemistry addition may be turned on again after the pressure reaches a certain point. By measuring pressure into a drinking line network; the dosing system will add chemistry, nothing or water accordingly. Dependent parameters are the volume of the drinking line network and expenditure rate.

The dosing of the biocide may occur at regular, timed intervals or it may occur at irregular intervals. The intervals may be preprogrammed into a water delivery system or they may be reactive based upon the water consumption by the animals as described above. The amount of biocide dosed into the medium or water may be predetermined or constant or it may fluctuate according to various feedback mechanisms. In an embodiment, dosing may include dosing for about 2 minutes up to about 45 minutes. In an embodiment irregular dosing may vary from 2 minutes up to about 45 minutes. In an embodiment, the interval of non-dosing or biocide-free interval may last from about 5 minutes up to about 10 hours, from about 15 minutes up to about 7 hours, from about 30 minutes up to about 5 hours, from about 45 minutes up to about 4 hours.

In an embodiment the biocide is dosed at a concentration of between about 0.01 and about 60 ppm by weight, between about 0.1 and 50 ppm by weight, between about 10 and 40 ppm by weight, and between about 20 and 35 ppm by weight. In an embodiment, the biocide is dosed at a relatively high concentration at about 30 ppm by weight. The biocide may initially be dosed at a higher or lower concentration including any amounts in the range of about 10 ppm to about 60 ppm. The dosing provided throughout a continuing cycle may vary or may remain constant.

Without being bound by theory, the method of the present invention is highly effective against micro-organisms as it uses a concentrated dose of peracetic acid or biocide to effectively kill, remove or prevent micro-organism build up or biofilm formation in the drinking lines, drinking nipples/cups and sediment. During a no treatment period, the overall water activity (Aw) lowers henceforth inhibiting or preventing micro-organisms from multiplying or growing. During the water rinse period, all present carbon sources (e.g. peracids) are removed, henceforth growth of moulds/yeasts and other micro-organisms that use peracetic acid residues as substrate are removed.

### EXAMPLES

A trial was conducted over a 39 day period to examine the effects of peroxyacetic acid and chlorine dioxide drinking water treatments on farm productivity and watering system hygiene at an egg-laying farm. The trial compared constant peroxyacetic acid and constant chlorine dioxide administration against interval dosed peroxyacetic acid administration.

The farm contained four hen-houses. Each house contained 6000 to 9500 hens. One house served as a control (no chemical was added to the water), while the other houses were used to test biocide treatments. Peroxyacetic acid (commercially available as *Incimaxx Aqua S*, from Ecolab Inc. located in St. Paul, MN having 10% peroxyacetic acid (POAA)) or chlorine dioxide (commercially available as *Selectrocide*, available from Selective Micro Technologies located in ______ having 5 grams ClO₂ per pouch).

ClO₂ stock solutions (1000 ppm) were prepared by adding *Selectrocide* pouches (contained sodium chlorite powder and acid powder) into 5 liters of water in air-tight containers. The stock solution was added to the poultry drinking water at 0.1%. The resultant levels of ClO₂ at the beginning and ending of the watering lines were 1 ppm and 0 ppm respectively.

*Incimaxx Aqua S* (*Incimaxx*) was added to the poultry drinking water at 0.02%. The resultant levels of POAA at the beginning and ending of the watering lines were 20 ppm and 10 ppm respectively. In a separate house, POAA was also tested using an intermittent or interval dosing regimen. In this regimen, birds were provided with drinking water containing *Incimaxx* at 0.03% for 15 minutes every 4 hrs.

All birds in the same hen house were the same age. However, the age of the birds from house-to-house varied. Table 1 lists the treatment and bird ages in each house.

**Table 1 - Summary of experimental units**

| **House number** | **Chemical** | **Age of hens at start of egg production** | **Age of hens at start of trial** |
|---|---|---|---|
| 1 | None | 115 days | 435 days |
| 2 | - Empty house - | | |
| 3 | *Incimaxx* | 124 days | 378 days |
| 4 | *Incimaxx* (interval) | 121 days | 194 days |
| 5 | ClO₂ | 128 days | 470 days |

Bird age was known to affect egg production. A brief overview of typical egg production cycles is provided below:
- Flock enters egg production at 126 to 154 days (10 to 20% of hens laying eggs)
- Flock reaches peak production at 210 to 224 days (~90% of hens laying eggs)
- Flock reaches "break-even" production at 420 to 490 days (~50% of hens laying)

Following the "break-even" point, the farmer has two options. These are, send the birds to a "spent hen" facility for processing or reduce the food and water for a few weeks to stimulate egg production. The follow-up egg production is relatively short-lived and does not reach previous levels.

For the reasons described above, egg production rates in the treatment groups were compared to historical data from the same age birds on untreated water.

The data collected during the trial included (1) daily water intake, (2) daily hen deaths, (3) daily egg production, (3) approximately weekly feed usage, (4) weekly bacteria counts from water samples from the end of the line, (5) weekly bacteria counts from watering system nipples, and (6) daily air temperature. Each of the results is detailed below except daily average temperature which was 19°C with very little day-to-day variation.

### Water hygiene

There was a general trend of lower total aerobic bacteria counts in drinking water and on drinker nipple surfaces; however, many reductions were not statistically significant (Tables 2 and 3 below). Coliform bacteria levels were unaffected by the chemical treatments as was expected. All three of the chemical treatments caused significant reductions in the numbers of yeasts and molds in the drinking water.

The reason for the mixed results is unknown but is attributed to the presence of bio-film on the watering line surfaces. This conclusion is based on the observed drop in active chemical levels between the start and end of the watering line.

The microbiological quality of the water exceeded hygiene specifications recommended by breeders and poultry scientists. Generally, <100 cfu/ml of total aerobic bacteria and <50 cfu/ml of coliform bacteria are recommended.

Reducing microbe levels in drinking water has been reported to reduce the energy expended by birds, presumably due to a reduction in immune system challenge. The reduced energy requirements may improve feed conversion.

**Table 2 - Microbe counts in drinking water collected from the end of the line (log CFU/ml)^{a}**

| **Treatment** | **Total aerobic bacteria*^{b}*** | **Coliform bacteria*^{b}*** | **Yeasts and molds*^{c}*** |
|---|---|---|---|
| Control | 5.34^{A} | 1.48^{A} | 2.61^{A} |
| *Incimaxx* | 4.85^{AB} | 1.85^{A} | 0.72^{B} |
| *Incimaxx* (interval) | 4.07^{B} | 1.72^{A} | 0.25^{B} |
| ClO₂ | 4.34^{AB} | 1.52^{A} | 0.09^{B} |

| | | | |
|---|---|---|---|
| *^{a}* Results in a column followed by different letters are significantly different (P-value <0.05). *^{b}* Mean of weekly samples (n = 10) *^{c}* Mean of biweekly samples (n = 5) | | | |

**Table 3 - Microbe counts in on drinker nipples (log CFU/ml)^{a}**

| **Treatment** | **Total aerobic bacteria*^{b}*** | **Coliform bacteria*^{b}*** | **Yeasts and molds*^{c}*** |
|---|---|---|---|
| Control | 5.51^{A} | 0.14^{A} | 0.30^{A} |
| *Incimaxx* | 4.92^{AB} | 0.32^{A} | 0.78^{A} |
| *Incimaxx* (interval) | 5. 10^{AB} | 0.58^{A} | 1.41^{A} |
| ClO₂ | 4.12^{B} | 0.08^{A} | 0.64^{A} |

| | | | |
|---|---|---|---|
| *^{a}* Results in a column followed by different letters are significantly different (P-value <0.05). *^{b}* Mean of weekly samples (n = 10) *^{c}* Mean of biweekly samples (n = 4) | | | |

### Water intake

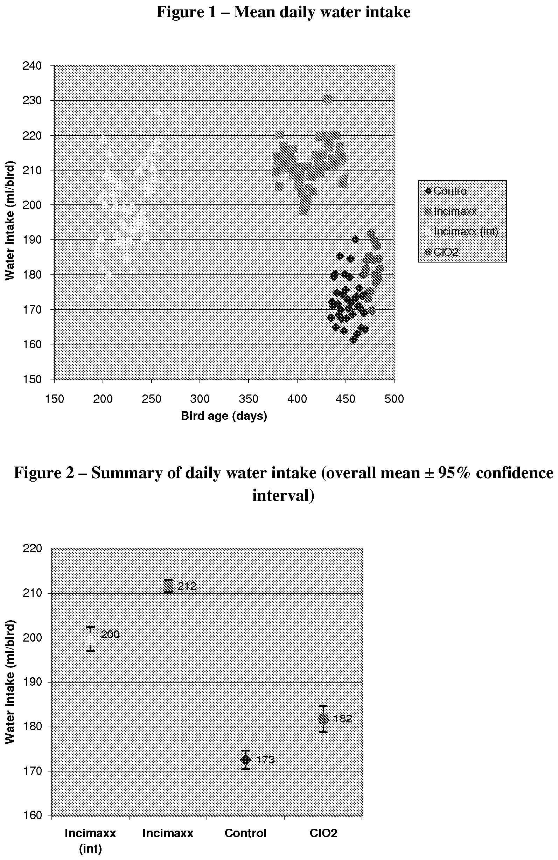

The birds provided with *Incimaxx-* or ClO₂-treated drinking water consumed significantly more water than the control birds (Figs. 1, 2). The increases in daily water consumption were 39 ml per bird (*Incimaxx*), 27 ml per bird (*Incimaxx* interval) and 9 ml per bird (ClO₂). Birds receiving *Incimaxx* by interval dosing showed greater variation in drinking water intake which was an expected result of the delivery system.

Chickens normally drink between 1.6 and 2.0-times their feed intake (by weight) of water on a daily basis at 21°C (70°F). Water consumption of more than 2 times the feed can occur in excessively high temperatures (above 30°C (86°F)).

The exact cause of the large increases in water consumption observed with the *Incimaxx* treatments is unknown. Possible explanations include bird age wherein younger birds are producing more eggs and thereby have greater nutritional requirements. Another explanation may be water acidity wherein a slightly acidic pH (e.g., pH 6) can stimulate water consumption. The treated water had a lower pH than the control water (although pH was not measured).

### Mortality rate

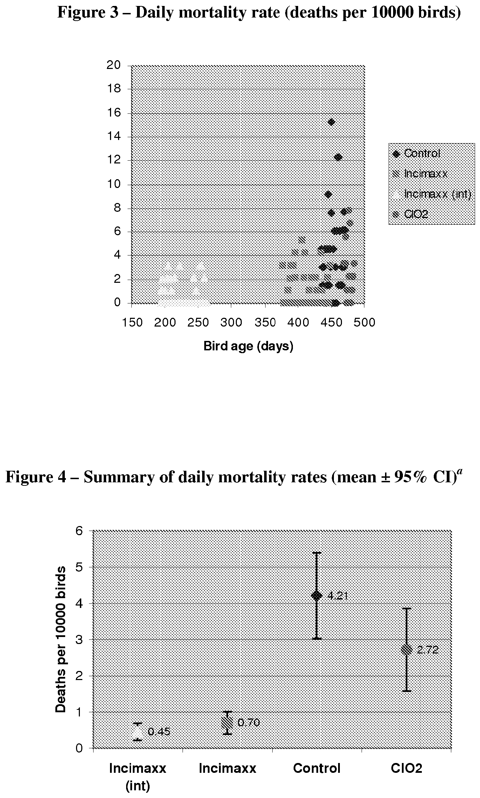

The daily mortality rates are presented in Figures 3 and 4. The birds provided with *Incimaxx-treated* drinking waters had significantly lower death rates than ClO₂-treatment and control birds. *Incimaxx* treatment reduced the daily death rate by 3.5 to 3.8 deaths per 10000 birds. Although birds in the control and ClO₂ groups were older, an increase in mortality is not expected among 250 to 450 day-old birds based on flock management guides provided by major poultry breeders (Cobb-Vantress, Aviagen, and Hubbard). The lower death rates among flocks in the *Incimaxx* treatment groups were presumably due to improved water hygiene.

### Egg production

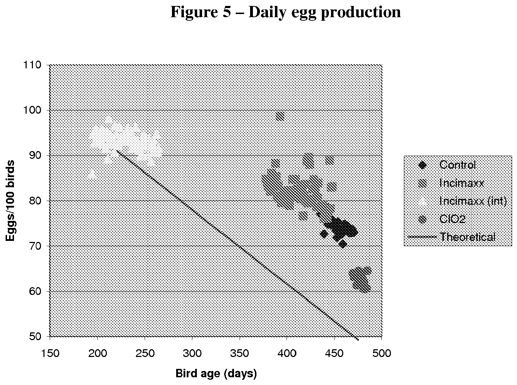

Egg production for the flocks in these studies was expected to peak at 90 eggs/100 birds when hens reached 220 days of age. After peaking, egg production rates were expected to follow a linear decline to 50% of peak production at 470 days. The actual egg production rates in all groups were higher than expected (Fig. 5). Results were not compared with historical data because it was unavailable.

### Feed conversion ratios

Greater feed intake rates in the *Incimaxx-treatment* groups were associated with greater egg output as shown in Table 4 below. Feed conversion ratios, that is, weight of feed per weight of product, for the chemical treatments were lower than the control indicating improved feed efficiency. It was surprisingly and unexpectedly found that the interval dosing provided the highest feed conversion rates. Therefore, not only was chemical consumption of the biocide reduced, but the output of eggs per feed input actually increased.

**Table 4 - Mean daily feed intake, egg production and feed conversion**

| **Treatment** | **Mean feed intake during study period (g feed/bird)** | **Mean egg production during study (egg/bird)** | **Feed conversion ratio (g feed/egg)** | **Feed conversion ratio (g feed/g egg)*^{a}*** |
|---|---|---|---|---|
| Control | 108 | 0.74 | 146 | 2.25 |
| *Incimaxx* | 116 | 0.83 | 140 | 2.15 |
| *Incimaxx* (interval) | 115 | 0.94 | 122 | 1.88 |
| ClO₂ | 80 | 0.63 | 127 | 1.95 |

| | | | | |
|---|---|---|---|---|
| *^{a}* Based on an assumed egg mass of 65 g for all treatments (note - egg mass not measured as part of this study) | | | | |

The invention has been described with reference to various specific and preferred embodiments and techniques. However, it should be understood that many variations and modifications may be made while remaining within the spirit and scope of the invention.

## Claims

1. A biocide comprising peracid for use in prophylaxis of infection, in particular for use in reducing and/or controlling the microbial population in the gastrointestinal tract of living animals, wherein the biocide is orally administered in an effective amount to an animal by interval dosing, wherein the interval dosing comprises administering orally between 10 to 60 ppm biocide for a time period of between 5 and 30 minutes supplied at between every 45 minutes to every 4 hours

2. The biocide for use as a medicament of claim 1, wherein the peracid comprises a peroxygenated carboxylic acid comprising performic, peracetic, perproprionic, peroxyheptanoic, peroxynonanoic, perlauric, monoperglutaric, diperglutaric, succinylperoxide, derivatives of perbenzoic acid, magnesium salt of peroxyphthalate, benzoyl peroxide, t-butylhydroperoxide, perlactic, percitric, perbutyric, peroctanoic, and perglycolic and combinations thereof.

3. The biocide for use as a medicament of claim 1, wherein the amount of the biocide administered is effective to reduce the population of microbes comprising Salmonella, Campylobacter, E. Coli, Listeria, and Helicobacter.

4. The biocide for use as a medicament of claim 1, wherein the animals comprise vertebrate animals.

5. The biocide for use as a medicament of claim 1, wherein the animals comprise poultry or cattle.

6. The biocide for use as a medicament of claim 1, wherein the step of administering the biocide comprises performing the step over a period just preceding slaughter.

7. The biocide for use as a medicament of claim 2 wherein the biocide further comprises sulfuric acid

8. The biocide for use as a medicament of claim 1, wherein the amount of the biocide compound administered is effective to kill microbes in the gastrointestinal tract.

9. The biocide for use as a medicament of claim 1, wherein the biocide is provided in drinking water.

## Patentansprüche

1. Biocid, das Persäure zur Verwendung bei einer Prophylaxe einer Infektion umfasst, insbesondere zur Verwendung bei einem Reduzieren und/oder einem Kontrollieren der mikrobiellen Population in dem Magen-Darm-Trakt lebender Tiere, wobei das Biocid einem Tier durch eine Intervalldosierung in einer wirksamen Menge oral verabreicht wird, wobei die Intervalldosierung das orale Verabreichen von 10 bis 60 ppm Biocid für einen Zeitraum von zwischen 5 bis 30 Minuten umfasst, das alle 45 Minuten bis alle 4 Stunden zugeführt wird.

2. Biocid zur Verwendung als ein Arzneimittel nach Anspruch 1, wobei die Persäure eine peroxygenierte Carbonsäure umfasst, die Folgendes umfasst: Perameisensäure, Peressigsäure, Perproprionsäure, Peroxyheptansäure, Peroxynonansäure, Perlaurinsäure, Monoperglutarsäure, Diperglutarsäure, Succinylperoxid, Derivate von Perbenzoesäure, Magnesiumsalz von Peroxyphthalat, Benzoylperoxid, t-Butylhydroperoxid, Permilchsäure, Percitronensäure, Perbuttersäure, Peroctansäure und Perglycolsäure und Kombinationen davon.

3. Biocid zur Verwendung als ein Arzneimittel nach Anspruch 1, wobei die Menge des verabreichten Biocids wirksam ist, um die Population von Mikroben zu reduzieren, die Salmonelle, Campylobacter, E. Coli, Listerien und Helicobacter umfassen.

4. Biocid zur Verwendung als ein Arzneimittel nach Anspruch 1, wobei die Tiere Wirbeltiere umfassen.

5. Biocid zur Verwendung als ein Arzneimittel nach Anspruch 1, wobei die Tiere Geflügel oder Rinder umfassen.

6. Biocid zur Verwendung als ein Arzneimittel nach Anspruch 1, wobei der Schritt des Verabreichens des Biocids ein Durchführen des Schritts über einen Zeitraum unmittelbar vor einer Schlachtung umfasst.

7. Biocid zur Verwendung als ein Arzneimittel nach Anspruch 2, wobei das Biocid ferner Schwefelsäure umfasst.

8. Biocid zur Verwendung als ein Arzneimittel nach Anspruch 1, wobei die verabreichte Menge der Biocidverbindung wirksam ist, um Mikroben in dem Magen-Darm-Trakt abzutöten.

9. Biocid zur Verwendung als ein Arzneimittel nach Anspruch 1, wobei das Biocid in Trinkwasser bereitgestellt wird.

## Revendications

1. Biocide comprenant un peracide destiné à être utilisé dans la prophylaxie d'une infection, en particulier destiné à être utilisé pour réduire et/ou lutter contre la population microbienne dans le tractus gastro-intestinal d'animaux vivants, le biocide étant administré par voie orale en une quantité efficace à un animal par introduction dosée par intervalle, dans lequel l'introduction dosée par intervalle comprend l'administration par voie orale d'entre 10 et 60 ppm de biocide pendant une période de temps comprise entre 5 et 30 minutes fourni entre toutes les 45 minutes et toutes les 4 heures.

2. Biocide destiné à être utilisé comme médicament selon la revendication 1, dans lequel le peracide comprend un acide carboxylique peroxygéné comprenant l'acide performique, peracétique, perproprionique, peroxyheptanoïque, peroxynonanoïque, l' acide perlaurique, monoperglutarique, diperglutarique, le peroxyde de succinyle, les dérivés d'acide perbenzoïque, le sel de magnésium du peroxyphtalate, le peroxyde de benzoyle, l'hydroperoxyde de t-butyle, l'acide perlactique, percitrique, perbutyrique, peroctanoïque et perglycolique et leurs combinaisons.

3. Biocide destiné à être utilisé comme médicament selon la revendication 1, dans lequel la quantité du biocide administré est efficace pour réduire la population de microbes comprenant Salmonella, Campylobacter, E. Coli, Listeria et Helicobacter.

4. Biocide destiné à être utilisé comme médicament selon la revendication 1, dans lequel les animaux comprennent les animaux vertébrés.

5. Biocide destiné à être utilisé comme médicament selon la revendication 1, dans lequel les animaux comprennent la volaille ou les bovins.

6. Biocide destiné à être utilisé comme médicament selon la revendication 1, dans lequel l'étape d'administration du biocide comprend la réalisation de l'étape sur une période juste avant l'abattage.

7. Biocide destiné à être utilisé comme médicament selon la revendication 2, dans lequel le biocide comprend en outre de l'acide sulfurique.

8. Biocide destiné à être utilisé comme médicament selon la revendication 1, dans lequel la quantité du composé biocide administré est efficace pour tuer les microbes dans le tractus gastro-intestinal.

9. Biocide destiné à être utilisé comme médicament selon la revendication 1, dans lequel le biocide est fourni dans de l'eau de boisson.
